# EUROPEAN PATENT SPECIFICATION

(11) **EP 1 458 568 B1**
(45) Date of publication and mention of the grant of the patent: **24.06.2009**
(21) Application number: 01992271.5
(22) Date of filing: 21.12.2001
(51) Int. Cl.: A61F 13/02, A61L 15/16

(54) **PROCESS FOR THE MANUFACTURE OF MULTI-LAYERED WOUND DRESSINGS**
VEFAHREN ZUR HERSTELLUNG VON MEHRLAGIGEN WUNDVERBÄNDEN
PROCEDE DE FABRICATION DE PANSEMENTS A MULTIPLES COUCHES

(43) Date of publication of application: 22.09.2004
(73) Proprietor: AVERY DENNISON CORPORATION, Pasadena, CA 91103 (US)
(72) Inventor: DE JONG, Johannes, H., A., Mentor, OH 44060 (US); LIPMAN, Roger, D., A., B-2360 Oud-Turnhou (BE); THOMAS, Patrick, B-2300 Turnhout (BE); NIJNS, Dirk, I., h., B-2340 Beerse (BE)
(74) Representative: Müller-Boré & Partner Patentanwälte
(86) International application number: PCT/US2001/049816
(87) International publication number: WO 2003/057485

(56) References cited:
- EP-A2- 0 320 814
- WO-A-01/60296
- US-A- 2 862 846
- US-A- 3 073 304
- US-A- 4 649 909
- US-A- 4 649 909
- US-A- 4 667 665
- US-A- 4 884 563
- US-A- 5 632 731

## Description

### Field of the Invention

The present invention relates to multilayered wound dressings suitable for the management of especially chronic wounds. More particularly, the invention relates to the manufacture of these dressings in a continuous process such that large quantities of individual dressings can be produced in a repeatable and cost efficient manner.

### Background of the Invention

Wound dressings, and in particular, multilayered wound dressings are known to be useful in speeding healing of acute wounds, stimulating healing of chronic non-healing wounds and in reducing wound pain. Wound dressings include transparent adhesive films, primarily constructed of polyurethane, non-transparent adhesives such as hydrocolloids, semi-transparent adhesives including hydrogels and non-transparent non-adhesives such as foams.

Hydrocolloid dressings have found widespread use especially for the management of chronic wounds, such as venostasis ulcers and decubitus ulcers. Hydrocolloid dressings also are useful for dressing certain acute wounds such as burns, donor sites and even post-surgical incisions. Hydrocolloid dressings are comprised of a suspension of fluid absorbent fillers in a pressure sensitive adhesive vehicle. Many hydrocolloid adhesive formulations have been described in the prior art. Suitable adhesive formulations may be found, for example, in the following patents, each of which is incorporated herein by reference in its entirety: US 3,339,546; US 4,231,369; US 4,367,732; US 4,477,325; US 4,738,257; US 4,551,490; US 4,192,785; US 4,952,618; WO 99/11728 and WO 99/14282.

Because the hydrocolloid is both adhesive and absorbent, these dressings can be placed in direct contact with the wound and can be adhered to the intact skin surrounding the wound. Generally they need no adjunct fixation and are thus convenient and economical to use. Hydrocolloid dressings are typically occlusive, which means they do not allow the wound to dry out and form eschar. Such dressings maintain the wound in a moist environment so that the cascade of cellular processes involved in wound healing proceeds in an optimal manner. Moreover, the moist hydrocolloid does not adhere to the wound. When a change of dressing is necessary the hydrocolloid dressing can be easily removed from the granulating wound bed without damaging the new tissues.

However, many chronic wounds are highly exudative, and one of the limitations of hydrocolloid dressings arises because of their relatively limited absorption capacity. If the dressing cannot absorb the wound exudate at a rate commensurate with exudate production by the wound, the dressing will quickly become saturated with fluid. This will cause the dressing to leak, and will cause maceration of the skin surrounding the wound. Wound exudate can be highly irritating to intact skin, and can cause the skin to break down and excoriate. Because these chronic wounds can often last for many months and even years, leakage of exudate can cause serious problems for the maintenance of healthy intact skin of the patient, and can therefore compromise effective wound management.

It is predominantly the older population that suffers from venostasis ulcers and decubitus ulcers. The market for dressings for these chronic wounds is therefore a growing one because of the changing demographics especially in developed countries. Considerable effort is being directed to development of improved chronic wound dressings, and this effort is very much directed to improve absorbency by use of composite dressing structures.

A number of approaches have been taken to develop dressings that have enhanced absorption capacity for chronic wounds. Dressings currently on the market that are indicated for heavily exuding wounds can comprise absorbent foams, such as absorbent polyurethane foams, or fibrous absorbents such as are used in dressings containing fibers of calcium alginate, or blends of sodium and calcium alginate, or fibers of sodium carboxymethyl cellulose. Examples of commercially available foam based dressings include Tielle^{®}, sold by Johnson & Johnson, Allevyn^{®}, sold by Smith & Nephew and Lyofoam^{®}, sold by the SSL Company. Examples of fibrous dressings currently on the market include Aquacel^{®}, sold by ConvaTec and Sorbsan^{®}, distributed by Maersk Medical.

However, there are drawbacks with the use of these foam and fiber based dressings. Neither material is inherently adhesive and therefore the fibrous or foam pad must be held in place with adjunct fixation. The Tielle^{®} product from Johnson & Johnson is available with the absorbent foam as an island pad centered on an adhesive coated microporous foam backing. While this dressing has a high capacity for fluid management, this capacity is achieved in part by transmission of water vapor through the microporous backing. Such water vapor transmission can lead to drying out of the wound, especially as the exudate level drops during the intermediate stage of wound healing. Drying of the wound can lead to significant delay in healing and even to deterioration of the wound bed by formation of eschar.

U.S. Patent 5,981,822 addresses this problem. This patent discloses a dressing comprising a wound contact sheet laminated to one side of an absorbent layer of water swellable material such as polyurethane foam. The wound contact sheet is provided with one or more slits. Expansion of the absorbent layer by exudate absorption causes the wound contacting sheet to expand, and the slits to open, thereby allowing passage of high flow rates of exudate. If the flow of exudate falls, then the slits close, thereby avoiding excessive drying of the wound bed.

There are many other examples of composite multilayered wound dressings in the prior art. U.S. Patent 4,793,337 discloses a composite dressing having an absorbent adhesive laminated to a fibrous absorbent such as an alginate. The fibrous absorbent is further laminated to a backing material such as a nonwoven polyester fabric by means of a second adhesive layer in between the fibrous layer and the nonwoven polyester fabric.

WO 00/41661 discloses a multilayered wound dressing having an absorbent layer having a high absorbency but a low lateral wicking rate and a transmission layer having a high moisture vapor transmission rate bonded to the side of said absorbent layer furthest from the wound. The wound dressing may also include an adhesive layer for adhering the dressing to the skin surrounding the wound. The optional adhesive may be a fluid interactive hydrocolloid adhesive and may be provided with perforations to assist transport of exudates through the dressing.

U.S. Patent 6,103,951 discloses a composite dressing having a polymeric cover layer bonded to a fibrous web that contains a mixture of a superabsorbent and a hydrocolloid. The fibrous web is characterized by a glazed surface of fused fibers on to which are deposited particles of hydrocolloid containing adhesive. The selvage edge of the dressing is fused to itself and to the polymeric cover layer. A process for the production of such a dressing is also described.

U.S. Patent 5,681,579 discloses a hydrocolloid having a polymeric support layer which can be continuous or discontinuous, an occlusive backing layer that overlies the support layer, an optional adhesive which may be a hydrocolloid adhesive on the skin contacting surface and an optional absorbent region interposed between the support and the backing layer.

EP 0 617 938 B1 discloses a wound dressing having a non-continuous hydrocolloid-containing polymeric support layer and an occlusive backing layer overlying the polymeric support layer, with an optional adhesive on at least a portion of the wound contacting surface, and an optional absorbent region interposed between the polymeric support and the occlusive layer.

U.S. Patent 5,968,001 discloses a wound dressing having a wound and skin contact layer, an upper occlusive layer with an absorbent layer in between with leak prevention seals which define an absorbent region. These seals disclosed as being effective in preventing the escape of wound exudate from the dressing on to the surrounding intact skin.

It can readily be seen from the foregoing review of the prior art that there has been considerable recent activity in the development of composite wound dressings suitable for chronic wound healing. In general, these composite wound dressings contain, as elements in their preferred embodiments, both hydrocolloid adhesives and another absorbent layer such as a fibrous or foam pad, the latter acting in such a composite as a sump to contain excess exudate. Moreover, composite absorbents of hydrocolloid and foam or fiber can also be backed with a microporous foam or film. The microporous foam or film backing will transport excess moisture out of the dressing but without the concomitant risk of drying the wound bed, since the hydrocolloid adhesive will maintain the wound bed moist even if the fibrous or foam absorbent becomes overly dry through evaporation.

The wound dressings described most recently in the prior art are much more complex than those earlier described and known wound dressings. Such complexity makes development of suitable high speed processes essential so that large quantities of these more complex dressings can be manufactured economically.

### Summary of the Invention

The present invention relates to a continuous manufacturing process for the production of complex multilayered wound dressings comprising laminates of several dissimilar materials in which the individual materials may be combined in various, predetermined configurations. The process of the present invention is a flexible, modular continuous manufacturing process, in which any or all of the unit operations including, for example, die cutting, lamination, island pad placement, heat bonding of composite absorbents, application of release liners, may be combined in different ways to manufacture specific dressing constructions. For example, the wound contact layer of the dressing may be die cut to form slits, perforations or apertures. The absorbent layer of the dressing may be applied as a discrete island of various dimensions to the wound contact layer. With the flexible process of the present invention, the dressing construction may be heat or adhesive bonded to give integrity in use. Further, a release liner having, for example, an unfolded, single fold or double fold configuration may be provided in the continuous process of the present invention.

In one embodiment, the present invention relates to combining some or all of the foregoing operations into a single, continuous process such that the individual materials may be continuously fed into the process, and the finished dressings continuously emerge at the end of the production line. In one embodiment, the steps are carried out in a registered continuous process.

Thus, the present invention relates to a process of manufacturing a multilayered wound dressing including an apertured wound contact layer, an absorbent material layer and a backing layer, including (a) providing a substantially continuous first web comprising the wound contact layer having a first and second major face and a first process release liner adhered to the first major face of the wound contact layer; (b) forming a plurality of apertures through the wound contact layer; (c) applying the absorbent material layer to the second major face of the wound contact layer; (d) laminating a substantially continuous web of the backing layer over the absorbent material layer to form the composite web; (d-1) removing the first process release liner and applying a product release liner, and (e) cutting the multilayered wound dressing from the composite web, in which the steps (a)-(e) are carried out in a continuous sequence.

In the circumstances of the present invention there has also been provided a method of manufacturing a multilayered wound dressing comprising an apertured wound contact layer, an absorbent material layer and a backing layer, including (a) providing a substantially continuous first web comprising the wound contact layer having a first and second major face, a first process release liner adhered to the first major face of the wound contact layer and the second process release liner adhered to the second major face of the wound contact layer; (b) forming a plurality of apertures through the wound contact layer by cutting, and removing the second process release liner and portions of the wound contact layer cut out from the apertures; (c) applying a discrete absorbent layer from a substantially continuous web of the absorbent material to the second major face of the wound contact layer; (d) laminating a substantially continuous web of the backing layer over the absorbent material layer to form the composite web; and (e) cutting individual multilayered wound dressings from the composite web, in which the steps (a)-(e) are carried out in a continuous sequence.

In the circumstances of the present invention there has been provided a further method of manufacturing a multilayered wound dressing comprising an apertured wound contact layer having a first and second major face, an absorbent material layer and a backing layer, including (a) providing a substantially continuous first web comprising the wound contact layer having a first and second major face and a first process release liner adhered to the first major face of the wound contact layer; (b) forming a plurality of apertures through the wound contact layer; (c) applying a discrete absorbent layer from a substantially continuous web of the absorbent material to the second major face of the wound contact layer, cutting and removing a portion of the web of the absorbent material; (d) laminating a substantially continuous web of the backing layer over the absorbent material layer to form the composite web; and (e) cutting the multilayered wound dressing from the composite web, in which the steps (a)-(e) are carried out in a continuous sequence.

Accordingly, the method of the present invention provides a process for preparing complex multilayer wound dressings in a continuous sequence.

### Detailed Description

The invention will now be more specifically described. In one embodiment, the present invention relates to a method of manufacturing a multilayered wound dressing comprising an apertured wound contact layer, an absorbent material layer and a backing layer, including steps of:
(a) providing a substantially continuous first web comprising the wound contact layer having a first and second major face and a first process release liner adhered to the first major face of the wound contact layer;
(b) forming a plurality of apertures through the wound contact layer;
(c) applying a discrete absorbent layer from a substantially continuous web of absorbent material to the second major face of the wound contact layer;
(d) laminating a substantially continuous web of backing layer over the absorbent material layer to form the composite web;
(d-1) removing the first process release liner and applying a product release liner, and
(e) cutting the multilayered wound dressing from the composite web, wherein the steps (a)-(e) are carried out in a continuous sequence.

A first embodiment of a multi-layered wound dressing made by a method in accordance with the present invention is explained. An individual, multi-layered wound dressing is representative of one type of dressing that may be manufactured using the method of the present invention. The wound dressing includes a backing layer, a layer of absorbent material interposed between the backing layer and a layer of fluid interactive adhesive. The layer of fluid interactive adhesive includes a plurality of apertures. The fluid interactive adhesive is protected by the product release liner, which is removed prior to use. As used herein, a fluid interactive adhesive is an adhesive that absorbs exudate, while at the same time adhering the dressing to the skin surrounding the wound. Such fluid interactive adhesives are disclosed, for example, in US Patent 4,538,603, which is hereby incorporated by reference.

One embodiment of the dressing is explained. Said embodiment is representative of one type of dressing that may be manufactured using the method of the present invention. The wound dressing includes the same elements as above, i.e., a backing layer, a selectively shaped absorbent material layer, an adhesive layer including a plurality of apertures, and further includes a two-part product release liner, i.e. a first and second release liner. Absorbent material layer is selectively bonded to a first major surface of the fluid interactive adhesive layer. This arrangement is completely covered by the backing layer. The second major surface of the fluid interactive adhesive layer is adhered to the first and second product release liners, which are removed prior to use.

An alternative embodiment of the multilayered wound dressing is further explained. This arrangement includes a backing layer that includes first and second extensions. The first and second extensions provide complete coverage of the adhesive layer with the backing layer. This arrangement helps to avoid contact with and adhesion to environmental contaminants such as dirt, dust, germs and other unwanted items that might otherwise become adhered to exposed portions of the adhesive.

In one embodiment, the dressings made according to the present invention are provided with a backing material that is generally occlusive, and in another embodiment may be of a porous construction such as a nonwoven fabric. Occlusive backings may be comprised of films or foams. Films such as polyurethanes and ethylene copolymers, for example, ethylene-vinyl acetate and ethylene-methyl acrylate are suitable, as are polypropylene films and polyvinylidene chloride films.

In one embodiment, the film thickness of the backing layer is in the range of from about 20 to about 500 microns. In another embodiment, the film thickness of the backing layer is in the range from about 40 to about 120 microns.

Examples of suitable foam materials are polyurethane foams, or polyethylene foams. The foam may be closed-cell or reticulated, or the cell structure may be intermediate between these two types. Film-foam laminates are also suitable. Foams of various thicknesses may be used. In general, foams up to about 1 mm thickness or more may be used. Nonwoven fabrics may be of any suitable construction.

The wound contact layer may be fabricated from any suitable material such as a polymeric or biological film, a foam, a net, a fabric or an adhesive. In one embodiment, the wound contact materials include one or more of polymeric films, polymeric nets and pressure sensitive adhesives. In another embodiment, the polymeric films include fluid interactive adhesives such as hydrocolloid adhesives, polyurethane pressure sensitive adhesives and hydrogel adhesives. In one embodiment, the wound contact layer is a hydrocolloid.

Hydrocolloid adhesives are generally first extruded or otherwise formed onto a silicone coated liner. In one embodiment the hydrocolloid is extruded or otherwise formed onto a glassine liner. This first liner performs the role of the top liner in the first step of the dressing manufacturing process. In one embodiment, the first liner has a thickness in a range from about 70 to about 160 microns, and in another embodiment, in the range from about 70 to about 90 microns. In one embodiment, the first liner has a release value in the range from about 40 and about 130 gm/25 mm width, and in another embodiment, in the range from about 60 to about 90 gm/25 mm width.

In one embodiment, a second liner is laminated over this construction. The second liner should be flexible enough to allow the total construction to be wound up with a smooth surface finish on the hydrocolloid. In one embodiment, the second liner has a release value that is not less than that of the first liner. In another embodiment, the release value of the second release liner is at least about 20 gm/25 mm width higher than the release value of the first liner. The second liner in one embodiment may be a film of high-density polyethylene with one or both of its sides siliconized. In one embodiment, the second liner may have a thickness in the range from about 50 to about 100 microns, and in another embodiment, a thickness in the range from about 60 to about 80 microns. In one embodiment, the second liner has a release value in the range from about 60 to about 120 gm/25 mm width, and in another embodiment, in the range from about 70 to about 100 gm/25 mm width.

Other liners may be utilised to replace either or both of the aforementioned liners so long as the relationship between the two liners and the tooling remains the same. By this it is meant that (a) the tooling is made to reflect the thickness of the liners used and (b) the differential release value between the liner on which the hydrocolloid adhesive is extruded (preferably the top liner in the dressing manufacturing process) and the liner that is laminated to the other side of the hydrocolloid is greater than zero and in one embodiment at least about 20 gm/25 mm width.

The absorbent material layer can be made of any material suitable for wound care that can absorb body fluids. Materials that may be used include fabrics, foams, fibrous structures of polyester, polypropylene, polyethylene and the like. Other suitable materials include as non-limiting examples natural and synthetic polymeric absorbents, superabsorbents and cellulosics. Fibrous absorbents manufactured from absorbent fibers such as alginate fibers and sodium carboxymethyl cellulose fibers, otherwise referred to as hydrofibers, are particularly useful. Composite materials that can direct the flow of the exudates, such as the blends of textile and gel-forming fibers laminated to a spreading layer of viscose polyester scrim described in WO 00/41661 may also be employed.

As used herein, the term "substantially continuous" means, with respect to a component of the process, for example the backing layer of the dressing, such component is provided in a long, continuous condition, such as on a supply roll, from which a plurality of parts may be obtained. The term "substantially" is included in recognition of the fact that a given supply roll must have a finite length. With respect to a process, the term "substantially continuous" is used in its conventional meaning, and means that the operation(s) is/are carried without significant interruption or cessation between steps.

The following is a general description of the process. The unit operations in the manufacturing process will be described in turn. The method of the present invention provides a substantially continuous method of manufacturing a multilayered wound dressing. In one embodiment, the steps are carried out in a registered continuous sequence, in which the individual steps are conducted in a single, continuous process and the steps are simultaneously performed in registration with each other and including substantially no breaks or accumulation of parts between the steps. The specific materials mentioned in the following description are exemplary only, and are provided by way of illustration, not of limitation.

Initially, there is explained an embodiment of an apparatus for carrying out the method of the present invention. In the first step, there is provided, from a supply roll, a substantially continuous first web which includes the wound contact layer and a first process release liner adhered to a first (or bottom) major face of the wound contact layer. In this embodiment, the first web includes a second process release liner adhered to a second (or top) major face of the wound contact layer. Suitable materials for the wound contact layer are disclosed above. In one embodiment, the first process release liner is high density polyethylene, and the second process release liner is glassine paper.

In the second step of the process a plurality of apertures are formed through the wound contact layer. In one embodiment, the apertures are die cut. The substantially continuous first web including the wound contact layer enters the first die cut station with the first process release liner on the bottom and the second process release liner on top. In one embodiment, the apertures are formed by kiss die cutting.

Apertures are cut into the wound contact layer in the web by means of a hardened steel rotary tool cutting against a hardened steel anvil roller, the web passing in between the tool and the anvil roller. As a result, aperture waste pieces are formed, which are then disposed of suitably. The tool is designed specifically to achieve selected and predetermined size, shape, and spatial arrangement of the apertures. The tool is further designed to cut through the first process release liner and the hydrocolloid, but not through the second process release liner.

In one embodiment, the cavities of the tool are filled with a plug material to prevent the aperture waste pieces from remaining in the tool. The plug material should be of a density that enables the plug to compress while under pressure and to return to its original shape following the release of the pressure. The thickness of the plug material depends on the tool design and should be such that it prevents the aperture of the first process release layer and the wound contact layer from remaining in the tool following the cutting of the aperture. The plug material may be made of any material deemed suitable, for example from polyethylene foam.

In one embodiment, the aperture waste pieces are removed from the web by removing the second process release liner to which the aperture waste pieces are adhered. The angle of delamination is, in one embodiment, between 0 and about 180°. In another embodiment, the angle is between about 10 and about 90°, and in another embodiment, the angle is between 20 and about 60°. In one embodiment, the differential release between both liners and the wound contact layer, disclosed above as between zero and about 20 gm/25 mm width, should be such that they overcome the tendency of the aperture waste to remain in the web. In other worlds, the release strength between the adhesive and the second process release liner should be sufficiently strong to assure that the aperture waste pieces are removed with the liner, while the wound contact layer remains adhered to the first process release liner. In one embodiment, an idler assists in controlling the angle of separation of the second process release liner from the web. The aperture waste pieces proceed, along with the top liner to a collection point.

If the wound contact layer is a material other than a fluid interactive adhesive, for example, a film wound contact sheet as specified in US Patent 5,981,822, means to establish an initial bond between the contact layer and the absorbent material layer may be required before proceeding to the next process step. In one embodiment, described below, the process includes a heating apparatus to accomplish this adhesion. In an alternate embodiment, the film wound contact sheet may be sprayed with, for example, a hot melt adhesive such as ethylene-vinyl acetate copolymer followed by compression with a heated roller to activate the adhesive.

Following the removal of the second process release liner and the aperture waste pieces the resultant web, which includes a selectively apertured wound contact layer and the first process release liner, the web proceeds to the absorbent layer application operation.

In one embodiment, the web passes around a pair of idlers. As noted above, the first idler assists in separation of the second process release liner from the web. The second idler assists in maintaining an optimum angle of approach to the second unit.

In the next step, the absorbent material layer is applied to the second major face of the wound contact layer. In one embodiment, this step is carried out at a second die cutting station. At the die cutting station, a continuous web of the absorbent material layer is applied to the first major face of wound contact layer and is cut into a predetermined shape. As noted above, in one embodiment, the absorbent material layer may be a hydrofiber material.

In one embodiment, a hardened steel rotary tool cuts the absorbent material from the top against a hardened steel anvil roller. In one embodiment, the tool has an angle of cut specific to material used for the absorbent material and a cutting depth specific to the material used in the wound contact layer on the bottom liner. In one embodiment, the rotary tool has been specifically shaped to achieve a predetermined shape of the absorbent material layer applied to the first major surface of the wound contact layer.

The rotary tool includes cavities which are defined by the cutting edges of the tool. The cavities and the cutting edges cut the absorbent material to a desired shape and size, and generate a waste matrix. As the web exits the second die station the waste matrix is removed. In one embodiment, removal of the waste matrix is assisted by passing the web around an idler. The waste matrix is conveyed to a removal system.

In one embodiment, the cavities of the tool are plugged in order to apply a small amount of pressure between the surface of the wound contact layer surface and the shaped absorbent material, encouraging it to remain in place during the removal of the matrix waste. In one embodiment, the plug material has a density in the range from about 60 to about 200 kg/m³. The density of the plug is selected to enable the plug to compress while under pressure (i.e., during the cutting and shaping of the absorbent material) and then to return to its original shape following the relief of the pressure.

Removal of the waste matrix leaves a web including specifically shaped and placed pieces of the absorbent material on the upper side of the layer of the specifically apertured wound contact material. In one embodiment, the absorbent material layer is applied to overlap the plurality of apertures in at least one area of overlap. Thus, in one embodiment, the absorbent material layer is a selectively sized and shaped, but relatively elongate sheet, which covers both the apertures and the remaining portions of the wound contact layer. The absorbent material layer and the wound contact layer are supported on the first process release liner. This resultant web then goes on to the next operation. In one embodiment, en route to the next processing station, the web passes around an idler. Said idler assists in orienting the web towards the third unit.

In the next, a substantially continuous web of the backing layer is laminated at a third die station over the absorbent material layer and the wound contact layer to form a composite web. The composite web includes the apertured wound contact layer, the absorbent material layer and the backing layer.

In one embodiment, the lamination is carried out by passing the web and the backing layer through the nip of a hardened steel anvil roller and a flexible rubber-covered steel laminating roller. The "rubber" covering may be a silicone rubber, high density polyethylene or any other suitable flexible plasticized polymer. In one embodiment, the flexible surface of the laminate roller has a hardness greater than about 50 Shore A, and in another embodiment, the hardness is above about 70 Shore A. In one embodiment, the laminating pressure is desirably kept to a minimum, for example, not more than 6 bar abs. In one embodiment, the laminate roller includes a relief structure along its peripheral surface.

In one embodiment, the backing layer, which may be a film or a foam as described above, is coated with a tie layer of adhesive. The adhesive may be, for example, a medical grade acrylic pressure sensitive adhesive. In one embodiment, the adhesive is the same adhesive used for the wound contact layer. In one embodiment, the backing layer may be supported on a removable liner described below. In one embodiment, the backing layer is unwound at the lowest possible tension, for example, less than about 20N. In another embodiment, the tension is less than about 10N. In an embodiment including a backing liner, the backing liner is removed in such a manner so as to avoid stretching of the unsupported film/foam layer prior to it being laminated.

In the next step of the process, the first process release liner of composite web is replaced with a product release liner. In one embodiment, the product release liner includes two parts.

In one embodiment, the first process release liner is removed from the composite web by passing the liner around an idler, having a predetermined diameter. In one embodiment, said idler has a diameter in the range from about 50 to about 80 mm. The idler facilitates the removal of liner. The resultant web then travels to a laminating nip; which includes a rubber covered steel pressure roller and a steel back-up roller, the product release liner(s) are introduced to the composite web. In one embodiment, the rubber covering the pressure roller has a hardness greater than about 50 Shore A, and in another embodiment, the hardness is greater than about 70 Shore A. In another embodiment, the laminate roller includes a relief structure along its peripheral surface. In one embodiment the liner replacement step further includes passing the product release liner(s) and the web through a nip defined by said rubber covered steel pressure and said steel back-up roller.

As noted above, the product release liners may be of any suitable design, for example one-piece or two-piece with one of the liners having a fold, to ease the removal of the said liner system by the end user. In one embodiment, a three-piece liner is used. The composite web, including the product release liner may be referred to as a second composite web.

The second composite web is passed from a laminating nip through a heating zone. The heating zone is comprised within a calendaring station. Alternatively, the heating step is carried out at another point in the process. In one embodiment, the heating step is omitted as unnecessary, because of the particular adhesive employed.

In said embodiment the second composite web enters the calendaring station by first passing around an idler, which assists in causing the web to wrap around a top roller. In one embodiment, the top roller is heated. The web passes around the top roller and then proceeds to pass through the nip between the top roller and a middle roller, and then around the middle roller. In one embodiment, the middle roller is heated. In one embodiment, both the top roller and the middle roller are heated. The second composite web passes around the middle roller and through the nip between the middle roller and a hardened steel anvil roller. In said embodiment, contact between the second web and the middle roller is maintained and assisted by an idler. As a result of passing through the calendaring station, the wound contact layer is bonded to the absorbent material layer in the at least one area of overlap between these layers.

In one embodiment, the heated roller has a predetermined diameter, and the heated roller has a different, also predetermined, diameter. In this embodiment, the difference in the predetermined diameters is controlled by hardened steel bearer rings. The difference in diameters creates an air gap between the two rollers through which the web passes. The specific air gap, temperature and speed of the web through the calendaring station enables the selection of specific bonding properties between the absorbent material layer and the first major surface of the wound contact layer.

In one embodiment, both the pressure at the nip between the top roller and the middle roller, which is defined and controlled by the gap in the nip, and the temperature of the rollers, are important to determining the bonding between the absorbent material layer and the wound contact layer. Of course, the nature of any adhesive used to bond these layers is also important. By selection and manipulation of these variables the nature of the bond between the absorbent material layer and the wound contact layer can be varied and controlled.

In one embodiment, the gap between the rollers is less than about 1 mm, and in another embodiment is between about 0.6 and about 0.9 mm. In general, the gap is fixed for a specific set of product components.

In one embodiment, the gap between the rollers, and the temperature of rollers are adjusted so that the pressure at the nip between the rollers is sufficiently high to drive the fibers of the absorbent material layer into a fluid interactive adhesive, such that fibers appear at the surface of the wound contacting layer. This embodiment produces a composite absorbent material that is able to wick wound exudate and is thereby able to increase the rate of exudate absorption. In this embodiment, forming apertures in the wound contacting surface is not necessary to achieve acceptable absorption.

In one embodiment, the operating temperature of the surface of the heated rollers, i.e. top roller and/or middle roller, is in the range from about 80 to about 130°C, and in one embodiment from about 90 to about 120°C. Dwell time, i.e., the time the second composite web is in contact with the heated surfaces, is defined by the line speed, the diameter of the rollers and the specific web path around them. In one embodiment, the dwell time is usually in the range from about one to about 15 seconds. The calendaring station is not limited as to the number of heated rollers. In various embodiments, from zero to three or more heated rollers can be incorporated in order to cooperate with the line speed for the other steps of the process, and hence throughput of the process of the invention may be controlled. In one embodiment, by appropriate selection of the adhesive, the calendaring station uses no heated rollers.

In the next process step, individual multilayered wound dressings are cut from the second composite web. In one embodiment, the dressing is cut from the composite web by die-cutting.

As the second composite web proceeds to this final cutting station, it passes through an outfeed nip formed by a pressure roller and a knurled roller before it is die cut. In another embodiment, the pressure roller is a rubber covered steel pressure roll. In one embodiments, the rubber covering the pressure roller has a hardness greater than 50 shore A, and in another embodiment, the rubber has a hardness of greater than 70 shore. In one embodiment, the pressure roller includes a relief structure along its peripheral surface. The outfeed nip helps to keep the web under tension and transport it to the cutting station.

In one embodiment, the final cutting is performed from above the web by a hardened steel rotary tool that is shaped specifically to the shape of the final patch of the multilayered wound dressing. The tool forms a nip and cuts against a hardened steel anvil roller. The patches of the multilayered wound dressing are delivered from the process on a conveyor belt. The resultant waste matrix is conveyed to a collection means.

The rollers of the first, second, third, final die cut stations, the heated calendaring station and the laminating station are all driven in a timed and synchronous manner that allows the die cutting and laminating required toachieve the multilayered wound dressings. The co-rotational action of these driven rollers transports the web through the machine. This timed relationship is adjustable for each individual station. The timing can be by any means deemed suitable.

In the following, several alternative embodiments are described. Where similar parts perform similar operations as described above with respect to the embodiment explained above, the description thereof is not repeated.

An alternative embodiment of the first die cut station is explained. As in the embodiment explained above, a web, including a wound contact layer, a first process release liner and a second process release liner, is provided to the first die cutting station. The die cut station includes a hardened steel anvil roll and a hardened rotary steel tool. The rotary tool is specifically shaped to form a desired shape in the aperture in the wound contact layer. The rotary tool cuts through the backing liner, through the hydrocolloid adhesive layer and through the thin release liner.

In said embodiment, the second process release liner is not removed with the aperture waste pieces. Instead, the aperture waste pieces are removed from the web by use of a counter roller and at least one pre-positioned blade. Two pre-positioned blades may be used. The counter roller may be made from aluminum, or may be made from suitable plastic materials, as well as from various metals. The counter roller includes a plurality of nodules on its outer surface. The nodules form a complimentary positive of the negative of the cavities in the rotary tool. The nodules are of a predetermined size slightly smaller than the aperture waste pieces. As the counter roller rotates the aperture waste pieces are pushed out by the nodules and are removed by the pre-positioned blades. The blades act to remove the waste pieces, for example, by scraping them off the web after the waste pieces are pushed through the web by the nodules.

There is explained a further alternative embodiment of the first die cut station. The die cut station comprises a hardened steel anvil roll and a hardened rotary steel tool as described above, in which the web is cut through its entire thickness, including through the first process release liner.

In said alternative embodiment, the aperture waste pieces are removed from the web by applying a jet of compressed air from a compressed air source to the web through a nozzle. The nozzle has a predetermined shape, size and internal taper, and the air has a predetermined pressure and flow rate to provide air of sufficient force to remove the aperture waste pieces from the web. A pair of idlers is arranged on either side of the nozzle at a predetermined distance apart. The idlers assist in removal of the aperture waste pieces by keeping the web under tension.

In addition to the foregoing means for removing the aperture waste pieces, other means for such removal known in the art may be used as appropriate.

There is now explained a further alternative embodiment of the process of the present invention which uses a radiant heating means in place of, or in addition to, the contact heating means described above. In this embodiment, the web is heated by at least one heat-emitting device, which is remote from the web. Thus the heat is not applied by contact heating, as in the calendaring station explained above. This heating method is particularly useful when the wound contact layer comprises a material other than a fluid interactive adhesive. For example, the wound contacting layer may comprise a film onto which an adhesive has been applied. This heating method may be used to activate the adhesive that has been applied to film. The heating mechanism can be infrared or any other means deemed suitable. The heating provides activation for the adhesive from which the wound contact layer is formed, and facilitates attachment of the absorbent material layer thereto. The subsequent process step is then carried out, substantially as described above. In another embodiment, the heating means is in direct contact with the web.

The resultant web is transported to a laminating station including a steel back-up roller and a laminating roller having a steel core and a rubber covering, substantially the same as in the embodiment explained above. As described above, the rubber covering may be, for example, a silicone rubber and may have a predetermined Shore hardness: The product release liner(s) are laminated to the web at the laminating station.

Having exited the laminating station the resultant web is transported to the final die cut station. The final die cut station comprises a knurled roller and pressure roll transporting the web to the hardened steel anvil roller and the hardened steel rotary tool. The rotary tool has been specifically shaped to cut the final multilayered wound dressing patch from the web.

In this embodiment, as in the embodiment explained above, the rollers of the first, second, third and final die cut stations, and the laminating station are all driven in a timed and synchronous manner that allows the die cutting and laminating required to achieve the multilayered wound dressings. The co-rotational action of these driven rollers transports the web through the machine. In this embodiment, as in all embodiments of the present invention, the timed relationship among the elements of the apparatus is adjustable for each individual station. The timing can be by any means known in the art that is deemed suitable.

According to a preferred embodiment, the wound contact layer is interposed between a first process release liner and a second process release liner. In one embodiment, the release liner has at least one of its sides siliconized, or otherwise treated to provide a similar release property. The release liner has a predetermined release property. In one embodiment, the second release liner has a predetermined release property that is lower than the predetermined release property of the first release liner. In one embodiment, the second release liner has at least one side siliconized or otherwise treated to provide a similar release property.

There is further an embodiment of the backing layer in which a removable release liner is adhered. In one embodiment, the backing liner includes an adhesive layer applied to the backing layer and covered by the release liner. The adhesive may be any known adhesive as appropriate to the present invention.

There is an alternative embodiment of the calendaring station. In this embodiment, the composite web enters the calendaring station by first passing around a pair of idlers, which assist in causing the web to wrap around a middle roller. In one embodiment, the middle roller is heated. As the web passes around the middle roller, it passes through a nip formed by the middle roller and a bottom hardened steel anvil roller. The web continues around the middle roller and then proceeds to pass through a nip between the middle roller and a top roller. The web then continues around the top roller. In one embodiment, the top roller is heated. In one embodiment, both the top roller and the middle roller are heated. The second composite web passes around the top roller and then around a series of idlers. Contact between the web and the rollers is maintained and assisted by the plurality of idlers.

The relationships between the top and middle rollers and the bottom hardened steel anvil roller, in particular, the relative diameters, the temperatures and the relative speeds of rotation are similar to those described above. The relationships between these parameters are similar, and the parameters may be similarly manipulated to control operation of the calendaring station.

While these particular embodiments of an improved method have been shown and described, it is to be understood that the invention is not limited only to these, and protection is sought to the extent defined by the following claims.

## Claims

1. A method of manufacturing a multilayered wound dressing comprising an apertured wound contact layer, an absorbent material layer and a backing layer, comprising:
(a) providing a substantially continuous first web comprising the wound contact layer having a first and second major face and a first process release liner adhered to the first major face of the wound contact layer;
(b) forming a plurality of apertures through the wound contact layer;
(c) applying a discrete absorbent material layer from a substantially continuous web of the absorbent material layer to the second major face of the wound contact layer;
(d) laminating a substantially continuous web of the backing layer over the absorbent material layer to form the composite web;
(d-1) removing the first process release liner and applying a product release liner; and
(e) cutting individual multilayered wound dressings from the composite web;
wherein the steps (a)-(e) are carried out in a continuous sequence.

2. The method of claim 1, wherein in step (b) the plurality of apertures are formed by die cutting.

3. The method of claim 2, wherein the first web further comprises a second process release liner adhered to the second major face of the wound contact layer.

4. The method of claim 3, further comprising simultaneously removing the second process release liner and portions of the wound contact layer die cut from the apertures.

5. The method of claim 2, wherein step (b) further comprises removing portions of the wound contact layer cut out from the apertures by applying an external force to the portions.

6. The method of claim 1, wherein the absorbent material layer is applied in step (c) to overlap the plurality of apertures in at least one area of overlap.

7. The method of claim 1, wherein the product release liner comprises a two-part release liner.

8. The method of claim 1, further comprising heating the composite web.

9. The method of claim 8, wherein the step of heating comprises contacting the composite web with at least one heated roller.

10. The method of claim 9, wherein the at least one heated roller is maintained at a temperature between about 80°C and about 130°C.

11. The method of claim 9, the composite web is contacted with at least two heated rollers.

12. The method of claim 8, wherein the step of heating comprises applying a remote heat source to heat the composite web.

13. The method of claim 8, wherein the heating step follows step (b).

14. The method of claim 8, wherein the heating step follows step (e).

15. The method of claim 1, wherein the step (e) further comprises passing the composite web through the nip of a knurled roller and a pressure roll.

16. The method of claim 1, wherein the wound contact layer comprises a fluid interactive adhesive.

17. The method of claim 16, wherein the fluid interactive adhesive is a hydrocolloid adhesive.

18. The method of claim 1, wherein the absorbent material layer is fibrous.

19. The method of claim 1, wherein the backing layer is coated with a pressure sensitive adhesive.

20. The method of claim 1, wherein the backing layer is an occlusive film or foam.

21. The method of claim 1, wherein the backing layer is microporous.

22. The method of claim 1, wherein the steps (a)-(e) are carried out in a registered continuous process.

23. The method of claim 1 wherein step (c) further comprises die cutting and removing the matrix of the continuous web of absorbent material.

## Patentansprüche

1. Verfahren zur Herstellung eines mehrschichtigen Wundverbandes, umfassend eine offene Wundkontaktschicht, eine Absorptionsmaterialschicht und eine Rückenschicht, umfassend:
(a) das Bereitstellen einer im wesentlichen kontinuierlichen ersten Bahn, umfassend die Wundkontaktschicht mit einer ersten und zweiten Hauptfläche und einen ersten Prozeßreleaseliner, anhaftend an die erste Hauptfläche der Wundkontaktschicht,
(b) das Bilden einer Vielzahl von Öffnungen durch die Wundkontaktschicht,
(c) das Aufbringen einer diskreten Absorptionsmaterialschicht von einer im wesentlichen kontinuierlichen Bahn der Absorptionsmaterialschicht auf die zweite Hauptfläche der Wundkontaktschicht,
(d) das Laminieren einer im wesentlichen kontinuierlichen Bahn der Rükkenschicht über die Absorptionsmaterialschicht, unter Bildung der Verbundbahn,
(d-1) das Entfernen des ersten Prozeßreleaseliners und das Aufbringen eines Produkt-Releaseliners, und
(e) das Ausschneiden von individuell mehrschichtigen Wundverbänden aus der Verbundbahn,
wobei die Schritte (a)-(e) in einer kontinuierlichen Sequenz durchgeführt werden.

2. Verfahren gemäß Anspruch 1, wobei in Schritt (b) die Vielzahl von Öffnungen durch Ausstanzen gebildet wird.

3. Verfahren gemäß Anspruch 2, wobei die erste Bahn weiter einen zweiten Prozeß-Releaseliner, anhaftend an die zweite Hauptfläche der Wundkontaktschicht, umfaßt.

4. Verfahren gemäß Anspruch 3, weiter umfassend das gleichzeitige Entfernen des zweiten Prozeß-Releaseliners und Abschnitten der Wundkontaktschicht ausgestanzt von den Öffnungen.

5. Verfahren gemäß Anspruch 2, wobei Schritt (b) weiter das Entfernen von Abschnitten der Wundkontaktschicht, ausgeschnitten von den Öffnungen durch Anwenden einer externen Kraft auf die Abschnitte, umfasst.

6. Verfahren gemäß Anspruch 1, wobei die Absorptionsmaterialschicht in Schritt (c) aufgebracht wird, die Vielzahl von Öffnungen in mindestens einem Überlappungsbereich zu überlappen.

7. Verfahren gemäß Anspruch 1, wobei der Produkt-Releaseliner einen zweiteiligen Releaseliner umfaßt.

8. Verfahren gemäß Anspruch 1, weiter umfassend das Erwärmen der Verbundbahn.

9. Verfahren gemäß Anspruch 8, wobei der Schritt des Erwärmens das Inkontaktbringen der Verbundbahn mit mindestens einer erwärmten Walze umfaßt.

10. Verfahren gemäß Anspruch 9, wobei die mindestens eine erwärmte Walze bei einer Temperatur zwischen etwa 80°C und etwa 130°C gehalten wird.

11. Verfahren gemäß Anspruch 9, wobei die Verbundbahn in Kontakt mit mindestens zwei erwärmten Walzen gebracht wird.

12. Verfahren gemäß Anspruch 8, wobei der Schritt des Erwärmens das Aufbringen einer abseitigen Wärmequelle zum Erwärmen der Verbundbahn umfaßt.

13. Verfahren gemäß Anspruch 8, wobei der Erwärmungsschritt dem Schritt (b) folgt.

14. Verfahren gemäß Anspruch 8, wobei der Erwärmungsschritt dem Schritt (e) folgt.

15. Verfahren gemäß Anspruch 1, wobei der Schritt (e) weiter das Leiten der Verbundbahn durch den Spalt einer Rändelwalze und einer Druckwalze umfaßt.

16. Verfahren gemäß Anspruch 1, wobei die Wundkontaktschicht ein Fluidinteraktives Haftmittel umfaßt.

17. Verfahren gemäß Anspruch 16, wobei das Fluid-interaktive Haftmittel ein Hydrokolloid-Haftmittel ist.

18. Verfahren gemäß Anspruch 1, wobei die Absorptionsmaterialschicht faserförmig ist.

19. Verfahren gemäß Anspruch 1, wobei die Rückenschicht mit einem Haftklebstoff beschichtet ist.

20. Verfahren gemäß Anspruch 1, wobei die Rückenschicht eine okklusive Folie oder Schaum ist.

21. Verfahren gemäß Anspruch 1, wobei die Rückenschicht mikroporös ist.

22. Verfahren gemäß Anspruch 1, wobei die Schritte (a)-(e) in einem registrierten kontinuierlichen Verfahren durchgeführt werden.

23. Verfahren gemäß Anspruch 1, wobei Schritt (c) weiter das Ausstanzen und Entfernen der Matrix der kontinuierlichen Bahn des Absorptionsmaterials umfaßt.

## Revendications

1. Procédé de fabrication d'un pansement à multiples couches comprenant une couche en contact avec la plaie ouverte, une couche de matériau absorbant et une couche de support, comprenant :
(a) la fourniture d'une première toile sensiblement continue comprenant la couche en contact avec la plaie, ayant une première et une seconde faces majeures et un premier revêtement anti-adhérent de processus adhérant à la première face majeure de la couche en contact avec la plaie ;
(b) la formation d'une pluralité d'ouvertures à travers la couche en contact avec la blessure ;
(c) l'application d'une couche de matériau absorbant discrète réalisée à partir d'une toile sensiblement continue de la couche de matériau absorbant à la seconde face majeure de la couche en contact avec la plaie ;
(d) la stratification d'une toile sensiblement continue de la couche de support sur la couche de matériau absorbant pour former la toile composite ;
(d-1) l'enlèvement du premier revêtement anti-adhérent de processus et l'application d'un revêtement anti-adhérent de produit ; et
(e) la découpe de pansements à multiples couches individuels de la toile composite ;
dans lequel les étapes (a)-(e) sont réalisées de façon continue.

2. Procédé selon la revendication 1, dans lequel dans l'étape (b), la pluralité d'ouvertures est formée par découpage.

3. Procédé selon la revendication 2, dans lequel la première toile comprend en outre un second revêtement anti-adhérent de processus adhérant à la seconde face majeure de la couche en contact avec la blessure.

4. Procédé selon la revendication 3, comprenant en outre l'enlèvement simultané du second revêtement anti-adhérent de processus et des portions de la couche en contact avec la blessure découpée des ouvertures.

5. Procédé selon la revendication 2, dans lequel l'étape (b) comprend en outre l'enlèvement de portions de la couche de contact avec la blessure découpée des ouvertures en appliquant une force externe aux portions.

6. Procédé selon la revendication 1, dans lequel la couche de matériau absorbant est appliquée dans l'étape (c) de façon à se superposer à la pluralité d'ouvertures dans au moins une zone de superposition.

7. Procédé selon la revendication 1, dans lequel le revêtement anti-adhérent de produit comprend un revêtement anti-adhérent en deux parties.

8. Procédé selon la revendication 1, comprenant en outre le chauffage de la toile composite.

9. Procédé selon la revendication 8, dans lequel l'étape de chauffage comprend l'entrée en contact de la toile composite avec au moins un galet chauffé.

10. Procédé selon la revendication 9, dans lequel ledit galet chauffé est maintenu à une température comprise entre environ 80 °C et environ 130 °C.

11. Procédé selon la revendication 9, la toile composite entre en contact avec au moins deux galets chauffés.

12. Procédé selon la revendication 8, dans lequel l'étape de chauffage comprend l'application d'une source de chaleur à distance pour chauffer la toile composite.

13. Procédé selon la revendication 8, dans lequel l'étape de chauffage suit l'étape (b).

14. Procédé selon la revendication 8, dans lequel l'étape de chauffage suit l'étape (e).

15. Procédé selon la revendication 1, dans lequel l'étape. (e) comprend en outre le passage de la toile composite à travers le rouleau moleté et un rouleau presseur.

16. Procédé selon la revendication 1, dans lequel la couche de contact avec la blessure comprend un adhésif interactif au fluide.

17. Procédé selon la revendication 16, dans lequel l'adhésif interactif avec le fluide est un adhésif hydrocolloïde.

18. Procédé selon la revendication 1, dans lequel la couche de matériau absorbant est fibreuse.

19. Procédé selon la revendication 1, dans lequel la couche de support est revêtue d'un adhésif sensible à la pression.

20. Procédé selon la revendication 1, dans lequel la couche de support est un film occlusif ou une mousse.

21. Procédé selon la revendication 1, dans lequel la couche de support est microporeuse.

22. Procédé selon la revendication 1, dans lequel les étapes (a)-(e) sont réalisées en un processus continu enregistré.

23. Procédé selon la revendication 1, dans lequel l'étape (c) comprend en outre le découpage et l'enlèvement de la matrice de toile continue en matériau absorbant.
